# EUROPÄISCHE PATENTSCHRIFT

(11) **EP 2 694 129 B1**
(45) Veröffentlichungstag und Bekanntmachung des Hinweises auf die Patenterteilung: **06.01.2016**
(21) Anmeldenummer: 12716310.3
(22) Anmeldetag: 05.04.2012
(51) Int. Cl.: A61M 1/36

(54) **TRAGE-, HALTE- UND SCHUTZVORRICHTUNG FÜR EXTRAKORPORALE HERZ- UND/ODER LUNGENUNTERSTÜTZUNGSSYSTEME**
SUPPORTING, RETAINING, AND PROTECTIVE APPARATUS FOR EXTRACORPOREAL HEART AND/OR LUNG SUPPORT SYSTEMS
DISPOSITIF DE SUPPORT, DE RETENUE ET DE PROTECTION POUR SYSTÈMES EXTRACORPORELS D'ASSISTANCE CARDIAQUE ET/OU PULMONAIRE

(30) Priorität: 08.04.2011 DE 102011016479
(43) Veröffentlichungstag der Anmeldung: 12.02.2014
(73) Patentinhaber: Universitätsklinikum Regensburg - Anstalt Des Öffentlichen Rechts, 93053 Regensburg (DE)
(72) Erfinder: PHILIPP, Alois, 93080 Pentling (DE); ARLT, Matthias, 61239 Ober-Mörlen (DE)
(74) Vertreter: Hannke, Christian
(86) Internationale Anmeldenummer: PCT/EP2012/056357
(87) Internationale Veröffentlichungsnummer: WO 2012/136808

(56) Entgegenhaltungen:
- US-A- 5 308 320
- US-A1- 2002 044 889
- US-A1- 2003 163 078
- US-A1- 2008 093 246
- US-A1- 2011 077 576

## Beschreibung

Die Erfindung betrifft eine Trage-, Halte und Schutzvorrichtung für extrakorporale Herz- und/oder Lungenunterstützungssysteme, insbesondere für eine Herz-Lungen-Maschine, mit einer geräteseitig angeordneten Mehrzahl an Halteeinrichtungen für das Festhalten von blutführenden und/oder blutbehandelnden Geräten und Steuerungseinrichtungen und mit mindestens einer patientenseitig angeordneten Befestigungseinrichtung zur lösbaren und schnellen Befestigung der Tragevorrichtung an einem Patienten oder einem in der Nähe des Patienten angeordneten Gegenstandes gemäß dem Oberbegriff des Patentanspruches 1, siehe z.B. US 2003/0163078.

Extrakorporale Herz- und/oder Lungenunterstützungsgeräte zur Aufrechterhaltung eines Blutkreislaufes, die in der Herzchirurgie eingesetzt werden, werden beispielsweise bei Herz-Lungen-Maschinen in tragbarer Form gewünscht. Derartige Geräte zur Aufrechterhaltung von beispielsweise einem extrakorporalen Blutkreislauf haben zur Aufgabe, dass beispielsweise zur Durchführung einer Perfusion im Rahmen der Chirurgie oder einer postoperativen Kreislaufunterstützung venöses Blut aus dem Körper eines Patienten aufbereitet werden muss, da die Herzfunktion eines Patienten während einer Operation unterbrochen bzw. reduziert ist. Dazu wird das venöse Blut in bekannter Weise so aufbereitet und mit Sauerstoff angereichert, dass es dem Kreislaufsystem eines Patienten zugeführt werden kann und dadurch alle notwendigen Organfunktionen während einer Operation aufrechterhalten werden können.

Herkömmlicherweise sind Vorrichtungen zur Aufrechterhaltung eines Blutkreislaufes sehr komplex aufgebaut. Es ist nicht nur sehr zeitaufwendig, eine derartige Vorrichtung an einem Patienten fachgerecht anzuschließen, sondern es ist auch das Fachwissen von erfahrenen Chirurgen und Kardiotechnikern notwendig, um eine derartige bekannte Vorrichtung funktionsgerecht zu betreiben.

Aus US 6,811,749 B2 ist eine Vorrichtung zur Aufrechterhaltung eines extrakorporalen Blutkreislaufes bekannt, die gegenüber den bekannten Vorrichtungen kompakter aufgebaut ist, da sie Teilkomponenten der Vorrichtung zusammenfasst und es dem Fachpersonal erleichtert wird, eine derartige Vorrichtung im Rahmen einer Herz-Bypass-Chirurgie mit einem Patienten zu verbinden. Diese kompakte Bauweise umfasst unter anderem ein Blutreservoire und einen Blutoxygenator. Das aus einem Patienten ausgeleitete venöse Blut strömt in das Blutreservoire und möglicherweise im System angereicherte Gasblasen werden in diesem Blutreservoire ausgeleitet, bevor das venöse Blut im weiteren Kreislauf mit Sauerstoff angereichert wird, damit es einem Patienten wieder zugeführt werden kann. Üblicherweise werden hierbei auch Blutpumpen verwendet.

Häufig weisen derartige Vorrichtungen zur Aufrechterhaltung eines Blutkreislaufes zwar eine kompakte Form auf, sind jedoch nicht für den tragenden Gebrauch, also für das schnelle Verlegen von einem Patientenbett zu dem anderen, oder für den Notfallbereich auf dem Land oder in der Luft, also direkt am Ort des Unfallgeschehens ausgelegt. Vielmehr sind derartige Vorrichtungen vorrangig für den Einsatz innerhalb von Krankenhäusern vorgesehen. Zudem sind sie schwer und unhandlich.

Es gibt zwar Vorrichtungen zur Aufrechterhaltung eines Blutkreislaufes, die auch in tragbarer Form ausgebildet sind. Allerdings ist hierbei nachteilhaft, dass die Vorrichtungen in ihrer Gesamtheit von einem einzigen Hersteller bzw. Anbieter angeboten werden. Es ist demzufolge nicht möglich, dass das klinische Personal bzw. der Arzt einen gewünschten Oxygenatortyp, Blutpumpentyp, hierfür notwendige Steuereinrichtungstypen, etc. sowie gewünschte Blutfiltertypen und Blasenfallen zur Befreiung des Blutes von Gaseinschlüssen individuell zusammenstellt.

Demzufolge ist es Aufgabe der Erfindung, eine Trage-, Halte und Schutzvorrichtung für extrakorporale Herz- und/oder Lungenunterstützungssysteme mit hohem Tragekomfort und Patientensicherheit für Vorrichtungen zur Aufrechterhaltung eines Blutkreislaufes zur Verfügung zu stellen, die vielseitig in Abhängigkeit von verschiedenen verwendeten Geräten auch als Schutzeinrichtung für diese Geräte ausgebildet sein und mobil und handlich eingesetzt werden kann.

Diese Aufgabe wird gemäß den Merkmalen des Patentanspruches 1 gelöst.

Ein wesentlicher Punkt der Erfindung liegt darin, dass bei einer Trage-, Halte und Schutzvorrichtung für extrakorporale Herz- und/oder Lungenunterstützungssysteme für Geräte zur Aufrechterhaltung eines Blutkreislaufes, insbesondere für eine Herz-Lungen-Maschine, mit einer geräteseitig angeordneten Mehrzahl an Halteeinrichtungen für das Festhalten von blutführenden und/oder blutbehandelnden Geräten und Steuerungseinrichtungen mit mindestens einer patientenseitig angeordneten Befestigungseinrichtung zum lösbaren und schnellen Befestigen der Tragevorrichtung an einem Patienten oder einem in der Nähe des Patienten angeordneten Gegenstandes zwischen den geräteseitigen Halteeinrichtungen und der patientenseitigen Befestigungseinrichtung mindestens ein schirmartiges Wandelement als Schutzelement für die Geräte angeordnet ist. Auf diese Weise ist nicht nur auf einfache Art eine Tragevorrichtung geschaffen, die einen Schutz gegenüber mechanischen und thermischen Schäden, die von der Patientenseite ausgehen können, für die Geräte zur Verfügung stellt, sondern es ist auch möglich, dass hierdurch eine Vielzahl von verschiedenen Geräten, wie beispielsweise Oxygenatoren, Blutpumpen sowie Steuerungseinrichtungen jeglicher Art, nämlich für temporäre oder langzeitextrakorporale Unterstützungssysteme jeglicher Art und Größe, auf einfach Weise angeordnet werden können. Es kann somit eine Kombination einer Vielzahl verschiedenster Geräte und Steuerungseinrichtungen ermöglicht werden, die zugleich schützend hinter dem bereits erwähnten Wandelement angebracht und mobil werden können. Hinzu kommt, dass dieses Wandelement gemäß dieser Tragevorrichtung nicht nur schützend, sondern auch einfach sowie leicht in seiner Bauweise ausgebildet sein kann. Derartige Haltevorrichtungen sind somit in Kombination mit dem Wandelement und den Befestigungseinrichtungen einfachst ausgebildet und schnell herstellbar und somit leicht in ihrem Gesamtgewicht, welches dafür entscheidend ist, dass diese Tragevorrichtungen mobil auf einfache Art und Weise schnell und in kompakter Form von einem Ort zu dem anderen eingesetzt werden können.

Die erfindungsgemäße Trage-, Halte und Schutzvorrichtung für extrakorporale Herz- und/oder Lungenunterstützungssysteme auch rückseitig, also nach hinten, mit mindestens einem Bandelement, vorzugsweise mehreren Wandelementen schützend versehen sein, so dass bei einem Passieren der Vorrichtung und im Falle eines Anstoßens durch eine Person an der Vorrichtung keine Schäden an den Geräten zur Aufrechterhaltung des Blutkreislaufes entstehen. Idealerweise sogar sämtliche Wandelemente, also vorderseitige und rückseitige Wandelemente sowie gegebenenfalls links- und rechtsseitig angeordnete Wandelemente zu einem geschlossenen Gehäuse mit einander verbunden, welches rückseitig und gegebenenfalls stattdessen oder zusätzlich vorderseitig oder auch zur Seite hin mit mindestens einer Schutzklappe oder einem türähnlichen Element versehen ist, um dieses geschlossene Gehäuse zu öffnen und die Geräte auswechseln zu können oder Verbindungsarbeiten zwischen den Geräten durchzuführen zu können. Anstatt von derartigen Wandelementen können selbstverständlich auch schutzgitterartige Elemente oder Schutzscheiben verwendet werden, wobei bei der Verwendung von Schutzscheiben denkbar ist, dass ähnlich wie bei der Verwendung von Visieren bei Motorradhelmen diese Schutzscheiben auf und zugeklappt werden können. Bei der Verwendung von Schutzgittern kann ein geschlossenes Gehäuse in Form eines Käfigs mit oder ohne aufklappbare Tür oder auch aufschiebbarer Tür entstehen. Sämtliche Gehäusevarianten können verschiedene Formen haben, wie beispielsweise eine Rechteckform.

Die Halteeinrichtungen sind demzufolge nicht nur derart ausgebildet, dass sie eine Vielzahl an Geräten, die auch standardmäßig auf dem Markt erhältlich sind, mit unterschiedlichen Größen umfassen, festhalten bzw. aufnehmen können, sondern auch eine offene und zugängliche Positionierung der Geräte und gegebenenfalls der Steuereinrichtungen einschließlich der damit zusammenhängenden Verbindungsschläuche für den Transfer von Blut und elektrische Verbindungen zur Verfügung stellen.

Das schirmartige Wandelement ist vorteilhaft zumindest teilweise in Form eines Abschnittes eines Zylindermantels ausgebildet.

Somit kann hinter dem schirmartig aufgebauten Wandelement eine Mehrzahl an Haltevorrichtungen, beispielsweise mindestens zwei flexibel einstellbare Haltevorrichtungen, angeordnet sein. Vorderseitig, also patientenseitig, ist mindestens eine Befestigungseinrichtung zum Befestigen der Tragevorrichtung an beispielsweise einem Notfallbett oder an einem anderen Gegenstand in der Nähe des Patienten oder dem Patienten selbst angebracht.

Gemäß einer bevorzugten Ausführungsform erstreckt sich eine Längsachse des Abschnittes des Zylindermantels des schirmartigen Wandelementes in im Wesentlichen vertikaler Richtung. Das Wandelement in Form eines Abschnittes eines Zylindermantels, welches beispielsweise die Hälfte eines vollumfänglichen Zylindermantels sein kann, sofern der Zylindermantel der Länge nach durchgeschnitten gedacht wäre, ist derart ausgerichtet, dass geräteseitig die konkav ausgebildete Seite des Abschnittes des Zylindermantels und patientenseitig die konvex ausgebildete Seite des Abschnittes des Zylindermantels angeordnet ist. Dies ermöglicht einen optimalen Schutz der Halteeinrichtungen durch den Zylindermantel, der sich halbkreisförmig - im Querschnitt betrachtet - um die Geräte und die Halteeinrichtungen, die dazugehörigen Steuereinrichtungen und Kabel sowie Blutverbindungstransportverbindungen legt. Zugleich ist es jedoch möglich, dass dieser Zylindermantelabschnitt mit halbkreisförmigem Querschnitt von oben und unten zugänglich ist und insbesondere von hinten zugänglich ist, um gegebenenfalls neue Geräte in den Halteeinrichtungen anzuordnen oder diese Geräte auszuwechseln sowie Korrekturen oder Installationen von Bluttransferleitungen und elektrischen Kabeln durchzuführen. Dies kann auf einfach Weise aufgrund dieser offenen Bauweise gestaltet werden, wobei zugleich ein optimaler Schutz von der Patientenseite aus für die Geräte möglich ist.

Selbstverständlich kann der Zylindermantel mit dem halbkreisförmigen Querschnitt auch ober- und unterseitig und auch gegebenenfalls von der Hinterseite, also von der Geräteseite aus, mit zusätzlichen Wandelementen abgedeckt sein, so dass ein komplett stützendes und die Geräte sowie restlichen Steuereinrichtungen vollständig umgebendes Gehäuse erhalten wird, um auch einen Schutz von der Rückseite, sowie von oben und unten zur Verfügung zu stellen.

Denkbar ist auch, dass das Wandelement nicht nur die Form eines Zylindermantels mit einem halbkreisförmigen Querschnitt, sondern auch eines Zylindermantels mit einem geringeren oder größeren Abschnitt eines Kreises in seinem Querschnitt darstellt. Alternativ können Wandelemente mit dreiecksförmigen, U-förmigen Querschnitten und dergleichen verwendet werden.

Durch derartig aufgebaute Wandelemente, vorzugsweise mit einem halbzylindermantelförmigen Querschnitt, besteht zudem ein guter Schutz, insbesondere ein mechanischer und thermischer Schutz, für den Patienten gegenüber den verwendeten Geräten. Dies erhöht die Sicherheit des Patienten gegenüber den eingesetzten Geräten im Falle einer Fehlfunktion oder dergleichen.

Alternativ zu der Verwendung eines Halbzylindermantels bzw. des schirmartig aufgebauten Wandelementes ist es auch denkbar, dass man stattdessen die mindestens zwei flexibel einstellbaren Haltevorrichtungen komplett mittels eines Umhüllungsbauteiles umhüllt. Dieses Umhüllungsbauteil kann selber als Haltevorrichtung mit mindestens zwei darin angeordneten Ausnehmungen zum Aufnehmen der verschiedenen Geräte ausgebildet sein, so dass dieses Umhüllungsbauteil zugleich Haltevorrichtung und Umhüllungsbauteil als Schutz gegenüber Verschmutzungen durch Blut oder dergleichen dient.

Materialien für ein derartiges Umhüllungsbauteil können beispielsweise Kunststoffmaterialien und/oder Styropor und/oder andere derartige Materialien in unterschiedlichen Farben sein.

Für die Anordnung eines derartigen komplett umhüllenden Umhüllungsbauteiles wird es möglich, dass ein schirmartig aufgebautes Wandelement integriert wird und hierdurch wiederum eine Tragevorrichtung mit geringem Gewicht leichter Handhabung, insbesondere im Notfalleinsatz, erhalten wird. Die Tragevorrichtung ist in sich leicht und multifunktionell gestaltet und ist vorzugsweise aus einem Einweg- oder einem Mehrweg-Werkstoff hergestellt. Dies bedeutet, dass bei Verwendung eines Einweg-Werkstoffes die Tragevorrichtung nach ihrem Einsatz, insbesondere bei Kontaktierungen mit Blut des Patienten, weggeworfen werden kann, ohne eine Reinigung hierfür erforderlich zu machen.

Alternativ kann bei Verwendung eines Mehrweg-Materials, wie beispielsweise karbonhaltiges Material, eine nach dem Einsatz im Notfall erfolgende Reinigung stattfinden, um die gesamte Tragevorrichtung aus hygienischen Gründen wieder komplett zu reinigen.

Es ist als Material ein Metall, welches oberflächenbeschichtet mit Kunststoff gegen thermische Abstrahlung ausgebildet ist, denkbar. Ebenso sind Kunststoffzusammensetzungen in verschiedenster Form denkbar.

Gemäß einer bevorzugten Ausführungsform weisen die Halteeinrichtungen Halteelemente auf, die in ihrer Geräteaufnahmegröße veränderbar sind, um Geräte mit unterschiedlichen Außenabmaßen aufnehmen zu können.

Derartige Halteelemente sind zumindest teilweise ringförmig und/oder zylinderförmig und/oder greifbackenförmig ausgebildet, um zumindest ein Gerät für das Festhalten und Aufnehmen zu umschließen.

Die Geräte können durchaus handelsübliche Geräte darstellen, wie beispielsweise handelsübliche Oxygenatoren oder Blutpumpen, an welche die Halteelemente passend genau oder universell passend anliegen, d.h. dass die Haltelemente, welche beispielsweise eine Art Ringelement oder zylindermantelförmiges Element sein können, derart eingestellt werdeh können, dass sie einen vorzugsweise zylinderförmigen Oxygenator oder eine zylinderförmige Blutpumpe passend genau aufnehmen können. Dies erhöht die Einsatzfähigkeit und kann im Notfall auch im freien Gelände eingesetzt werden, ohne dass hierbei ein Verrutschen der einzelnen Geräte innerhalb der Tragevorrichtung stattfindet. Die Beschädigung der Geräte wird hierdurch weitestgehend vermieden. Ebenso kann hierdurch vorteilhaft eine beliebige Kombination von Geräten, sofern dies der Arzt bzw. der Anwender wünscht, durchgeführt und realisiert werden, um nicht nur Geräte von einem Hersteller, sondern Geräte verschiedenster Hersteller benutzen zu können. Es ist somit eine individuell zusammengesetzte Herz-Lungen-Maschine realisierbar. Auch können notwendige Zusatzgeräte wie z. B. eine Sauerstoff- oder Druckluftflasche, ein Beatmungsgerät, ein Monitor oder Defibrillator verwendet werden.

Selbstverständlich können die Halteelemente an ihren freien Enden irgendeine Art von Zwinge, Haken, Greifelement oder dergleichen aufweisen, um die Geräte der Tragevorrichtung schnell und einfach auswechseln zu können.

Derartige Befestigungen mittels Zwingen, Haken, Greifelementen, Gurten oder dergleichen können auch bei der Befestigungseinrichtung angewendet werden, die eine schnelle, einfach sowie vorübergehend oder dauerhafte Befestigung der Tragevorrichtung an dem Bett oder einem (Luft-)Fahrzeug oder Gegenstände dergleichen ermöglichen soll. Die Befestigungseinrichtung soll universell ausgestaltet sein und eine nahezu uneingeschränkte Mobilität von Tragevorrichtungen für die Aufrechterhaltung eines Blutkreislaufes ermöglichen. Die Befestigung kann auch Patientenfem notwendig sein z.B. bei der Verwendung von O₂-Flaschen oder Steuerkonsolen.

Die Halteeinrichtungen sind vorzugsweise - in horizontaler Richtung betrachtet - nebeneinander angeordnet, so dass ein geordnetes Positionieren der Geräte und ein leichtes Miteinander-Verbinden dieser Geräte untereinander und mit den Steuereinrichtungen möglich ist. Denn durch das Nebeneinander-Anordnen der Halteeinrichtungen und somit der Geräte können die Geräte von oben und von unten zugänglich gemacht werden, um derartige Verbindungen herzustellen.

Beispielsweise ist die Zu- und Ableitung für den Blutfluss zu und von der Trage-, Halte und Schutzvorrichtung für extrakorporale Herz- und/oder Lungenunterstützungssysteme von oben kommend gestaltet. Dies bedeutet, dass sowohl die Zuleitung als auch die Blutableitung von oben zu der Tragevorrichtung geleitet werden, also beispielsweise bei der Verwendung des zylindermantelförmigen Wandelementes über die Stirnseite des Abschnittes des Zylindermantels zu den Geräten geleitet werden.

Eine Blutflussmesseinrichtung kann auf dem Weg von den bluttransferierenden Geräten zu einer Steuereinrichtung innerhalb der Tragevorrichtung um einen Bluttransferschlauch gelegt werden und dieser Blutflussmesser sendet somit vorzugsweise drahtlos Daten über eine Blutflussmessung mittels Impedanzmessung. Alternativ oder zusätzlich zu der Blutflussmesseinrichtung kann jeder andere Art von Messeinrichtung verwendet werden, wie beispielsweise eine Messeinrichtungfür die Messung der Sauerstoffsättigung, einer Temperatur des Blutflusses oder der Messung eines HB-Wertes.

Die Halteeinrichtungen sind vorzugsweise mittels mindestens eines Haltesteges an der konkav ausgebildeten Seite des schirmartigen Wandelementes befestigt, wobei in dem Haltesteg oder zwischen dem Haltesteg und der Halteeinrichtung und/oder dem Wandelement mindestens ein Gelenk, vorzugsweise ein Kugelgelenk, oder eine Vielzahl von Gelenken, Scharnieren und/oder Verstelleinheiten, die in verschiedene Richtungen betätigbar sind, angeordnet ist, um die Halteeinrichtungen in ihrer gegenseitigen Ausrichtung zueinander zu verändern. Auch kann mindestens eine um ihre Langachse axial drehbare Haltestange verwendet werden. Hierdurch wird nicht nur ein besseres und vorteilhafteres Anordnen der einzelnen Geräte mit in Abhängigkeit von ihren Außenabmaßen unterschiedlichem Bedarf innerhalb der Tragevorrichtung möglich, sondern auch ein leichteres und schnelleres Verbinden der Geräte untereinander mittels Verbindungsleitungen und dergleichen möglich. Zudem kann ein übersichtliches Anordnen der einzelnen Geräte und damit der Halteeinrichtung sowie von Steuereinrichtungen ermöglicht werden.

Die Befestigungseinrichtung oder zwischen der Befestigungseinrichtung und der konvex ausgebildeten Seite des Wandelementes kann ebenso mit mindestens einem Gelenk, vorzugsweise einem Kugelgelenk, oder eine Vielzahl von Gelenken, Scharnieren und/oder Verstelleinheiten, die in verschiedene Richtungen betätigbar sind, ausgestattet sein, um das Wandelement und die Halteeinrichtungen gegenüber dem Gegenstand, wie einem Patientenbett oder dem Patienten selbst, auszurichten. Auch kann mindestens eine um ihre Langachse axial drehbare Haltestange verwendet werden. Somit kann eine Kopplung der Tragevorrichtung unter Bezugnahme auf den Gegenstand, an dem die Tragevorrichtung befestigt werden soll, geneigt werden oder zur Seite hin geschwenkt werden, so dass eine nicht störende und vorteilhafte Positionierung der gesamten Tragevorrichtung möglich ist. Dies ist insbesondere in Notfalleinsätzen sehr wichtig, wo ein geringer Platz in den Notfallfahrzeugen zur Verfügung steht.

Gemäß einer bevorzugten Ausführungsform sind die Halteeinrichtungen derart ausgestaltet, dass mit ihnen Oxygenatoren, Blutpumpen, Blasenfallen sowie Steuerungseinrichtungen für temporäre oder langzeitextrakorporale Unterstützungssysteme jeglicher Art und Größe umfassbar oder fest haltbar sind.

Das Wandelement ist auf Höhe der Kontaktpunkte der Haltestege mit der konkav ausgebildeten Seite des Wandelementes mit zumindest einem teilweise kreisförmigen Versteifungselement ausgestattet, so dass eine Stabilität des relativ dünnwandigen Wandelementes des Zylindermantels zumindest teilweise vorliegt und eine stabile Anordnung der Haltestege und damit der Halteeinrichtungen an diesem Wandelement möglich ist.

Eine derartige Tragevorrichtung kann vorteilhaft sowohl bei boden- als auch bei luftgebundenem Transport jeglicher Art eingesetzt werden und ist sowohl für öffentliche als auch für private Räume, medizinische Behandlungseinheiten jeglicher Art sowie innerhalb und außerhalb des Krankenhauses geeignet. Der Grund für die kompakte Anordnung der einzelnen Geräte an der Tragevorrichtung ist schnelles Wechseln der Tragevorrichtung von einem Ort zu dem nächsten und somit eine hohe Mobilität der Tragevorrichtung auch in Hinblick auf den zu transportierenden Patienten, beispielsweise von dem Ort des Noteinsatzes zu dem Krankenhaus.

Weitere vorteilhafte Ausführungsformen ergeben sich aus den Unteransprüchen.

Vorteile und Zweckmäßigkeiten sind der nachfolgenden Beschreibung in Verbindung mit der Zeichnung zu entnehmen. Hierbei zeigen:
- Fig. 1: in einer schematischen Darstellung eine Draufsicht auf eine Tragevorrichtung gemäß einer Ausführungsform der Erfindung mit angedeutetem Krankenbett; und
- Fig. 2: in einer schematischen Darstellung die erfindungsgemäße Tragevorrichtung gemäß der Ausführungsform nach Fig. 1 in einer Drückansicht.

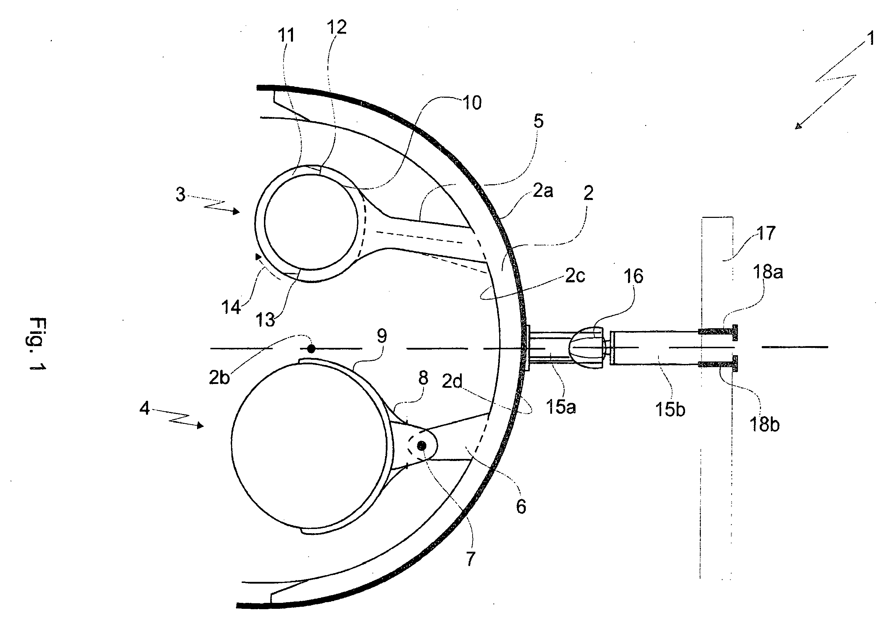

Fig. 1 ist in einer schematischen Darstellung die Tragevorrichtung gemäß einer Ausführungsform der Erfindung als Draufsicht wiedergegeben. Dieser Darstellung ist zu entnehmen, dass die Tragevorrichtung 1 aus einem im Querschnitt halbkreisförmigen zylindermantelförmigen Wandelement 2 besteht, welches außenseitig mittels einer zusätzlichen Oberflächenschicht 2a eine thermische Schutzschicht aufweist. Dies betrifft die konvex ausgebildete Seite des Wandelementes 2.

Die konkav ausgebildete Seite 2c des Wandelementes ist mit Halteeinrichtungen 3, 4 ausgestattet, die dazu dienen, Geräte verschiedenster Art für den Aufbau einer Herz-Lungen-Maschine aufzunehmen.

Das Wandelement 2 erstreckt sich zylindermantelförmig entlang einer Längsachse 2b, weist also eine Zylindermantellängsseite in die Bildebene hinein betrachtet auf.

Die Halteeinrichtungen 3, 4 weisen verschiedenartige Haltelemente auf und sind mittels Haltestegen 5, 6, die ebenso verschiedenartig ausgebildet sind, an der konkav ausgebildeten Seite 2c angeordnet.

Beispielsweise ist der Haltesteg 6 mittels eines Gelenkes 7, welcher eine nach unten gehende bzw. in die Bildebene hineingehende Achse aufweist, mit einem Abschnitt 8 eines Halteelementes 9 verbunden, um so das Haltelement 9 und damit die Halteeinrichtung 4 gegenüber dem Wandelement 2 verschwenkt ausbilden zu können.

Der Haltesteg 5 der Halteinrichtung 3 hingegen besteht aus einem nicht veränderbaren festen Steg und stellt die Verbindung zu den Haltelementes 10, 11, 12 und 13 her.

Das Haltelement 11 ist gegenüber dem Halteelement 10 aufklappbar, wie es beispielsweise durch den Pfeil 14 wiedergegeben wird. Durch dieses Aufklappen kann um ein hier nicht näher dargestelltes Scharnier im Bereich des Bezugszeichens 12 ein zylinderförmiges Gerät, wie beispielsweise ein Oxygenator, in die Halteeinrichtung 3 aufgenommen werden und anschließend wieder zugeklappt werden. Nach erfolgtem Zuklappen findet ein Verschließen im Bereich des Bezugszeichens 13 statt.

Eine Befestigungseinrichtung aus den beiden Elementen 15a und 15b ist mit einem Kugelgelenk 16 unterbrochen und ermöglicht ein Verschwenken der restlichen Tragevorrichtung, also des Wandelementes 2 zusammen mit den Halteeinrichtungen 3, 4 gegenüber einem Gegenstand 17, welcher der Rand eines Patientenbettes sein kann, in jede Richtung. Dies bedeutet, dass das Wandelement 2 einschließlich der Halteeinrichtungen 3, 4 nach oben, nach links, nach rechts, nach unten oder jegliche dazwischen angeordnete Winkel verschwenkt werden kann, um ein optimiertes Positionieren der Tragevorrichtung gegenüber dem Bett beispielsweise bei gering vorhandenem Platz zu ermöglichen.

Der Rand 17, der hier lediglich angedeutet ist, eines Patientenbettes wird oberseitig von zwei Greifarmen 18a und 18b, die an der Befestigungseinrichtung 15a, 15b befestigt sind, umfasst und ermöglicht ein schnelles Einhaken der Tragevorrichtung an dem Patientenbett.

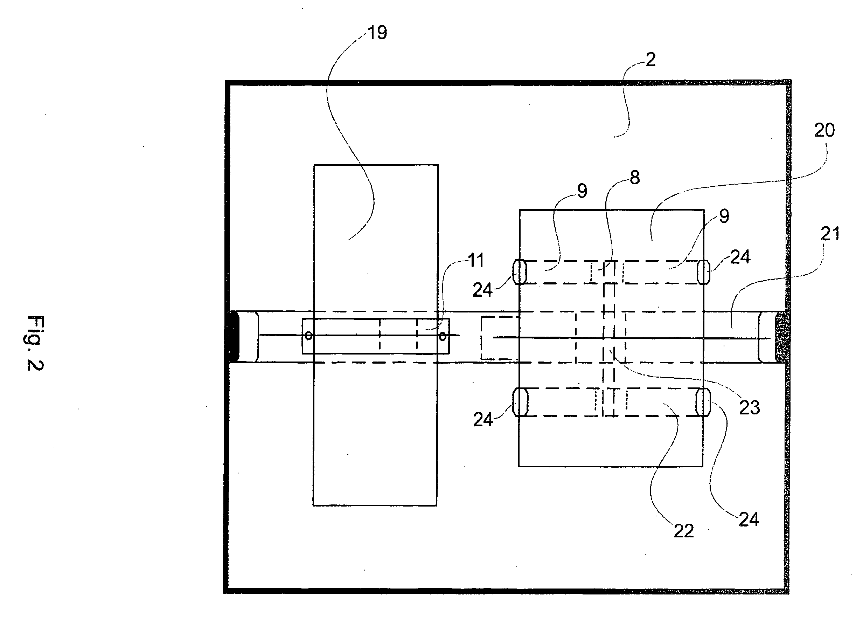

In Fig. 2 ist in einer Rückansicht die erfindungsgemäße Tragevorrichtung gemäß der Ausführungsform nach Figur 1 dargestellt. Dieser Darstellung ist zu entnehmen, dass hinter dem Wandelement 2, also auf geräteseitiger Seite, die Halteeinrichtungen 3, 4 mit den Halteelementen 11, 9 und 22 nebeneinander angeordnet sind. Vorteilhaft sind beide Halteeinrichtungen an einem gemeinsamen Versteifungselement 21, welches sich entlang der Innenseite des zylindermantelförmigen Wandelementes 2, also entlang der konkav ausgebildeten Seite, erstreckt, verbunden. Diese Verbindung kann mittels einer Schweißverbindung oder dergleichen erfolgt sein. Auf diese Art ist eine feste Verbindung zwischen dem Wandelement 2 und Haltestegen der Halteeinrichtung 3, 4 sichergestellt.

Zwei Geräte 19, 20 sind innerhalb der Halteeinrichtungen 3, 4 angeordnet.

Der Darstellung ist ebenso deutlich zu entnehmen, dass das recht Gerät 20 nicht nur mittels eines oberen ringförmig ausgebildeten Elementes 9 sondern auch mittels eines unteren ringförmig ausgebildeten Halteelementes 22 gehalten wird.

Die ringförmigen Haltelemente 9, 22 sind nicht vollständig geschlossen, sondern sind rückseitig, also aus der Bildebene heraus, offen gestaltet, so dass ein schnelles Zuführen und wieder Abnehmen des Gerätes 20 möglich ist.

Vorzugsweise sind die Halteelemente 9, 22 unter Federspannung auseinanderdrückbar, also in Bildebene betrachtet nach links und nach rechts auseinanderdrückbar, so dass ein Gerät 20 schnell und einfach darin eingeklemmt werden kann. Hierfür weisen die Halteelemente 9, 22 endseitig Schutz- und Gleitbacken 24 auf, die zum schnellen Zu- und Abführen ohne Beschädigung des Gerätes 20 dienen sollen.

Ein Verbindungselement 23 verbindet die beiden Halteelemente 22 und 9, um von diesem Verbindungselement 23 auch etwa halber Höhe weggehend einen Haltesteg, der hier nicht näher dargestellt ist, zu dem Versteifungselement 21 in nahezu horizontaler Richtung verlaufen hingehen zu lassen.

Sämtliche in den Anmeldungsunterlagen offenbarte Merkmale werden als erfindungswesentlich beansprucht, sofern sie einzeln oder in Kombination gegenüber dem Stand der Technik neu sind.

### Bezugszeichenliste

- 1: Tragevorrichtung
- 2: Wandelement, Längsachse, Zylindermantel
- 2a: Oberflächenschicht, Zylindermantel
- 2b: Längsachse
- 2c: konkav ausgebildete Seite (des Wandelementes)
- 2d: konvex ausgebildete Seite
- 3: Halteeinrichtung
- 4: Halteeinrichtung
- 5: Haltesteg
- 6: Haltesteg
- 7: Gelenk
- 8: Abschnitt
- 9: Halteelement
- 10: Halteelement
- 11: Halteelement
- 12: Halteelemente
- 13: Halteelement
- 14: Pfeil
- 15a: Element
- 15b: Element
- 16: Befestigungseinrichtung
- 17: Gegenstand, Rand, Patientenbett
- 18a: Greifarm
- 18b: Greifarm
- 19: Gerät
- 20: Gerät
- 21: Versteifungselement
- 22: Halteelement
- 23: Verbindungselement
- 24: Schutz- und Gleitbalken

## Patentansprüche

1. Trage-, Halte und Schutzvorrichtung für extrakorporale Herz- und/oder Lungenunterstützungssysteme insbesondere für Geräte zur Aufrechterhaltung eines Blutkreislaufes, wie eine Herz-Lungen-Maschine, mit einer geräteseitig angeordneten Mehrzahl an Halteeinrichtungen (3, 4) für das Festhalten von blutführenden und/oder blutbehandelnden Geräten (19, 20) und Steuerungseinrichtungen,
und mit mindestens einer patientenseitig angeordneten Befestigungseinrichtung (15a, 15b, 16) zum lösbaren und schnellen Befestigen der Tragevorrichtung (1) an einem Patienten oder einem in der Nähe des Patienten angeordneten Gegenstandes (17),
**dadurch gekennzeichnet, dass**
zwischen den geräteseitigen Halteeinrichtungen (3, 4) und der patientenseitigen Befestigungseinrichtung (15a, 15b, 16) mindestens ein schirmartiges Wandelement (2, 2a) als Schutzelement für die hinter dem Wandelement angebrachten Geräte (19, 20) angeordnet ist, welches zumindest teilweise die Form eines Abschnittes eines Zylindermantels aufweist, wobei sich eine Längsachse (2, 2b) des von hinten zugänglichen Zylindermantels (2, 2a) in vertikaler Richtung erstreckt.

2. Trage-, Halte und Schutzvorrichtung für extrakorporale Herz- und/oder Lungenunterstützungssysteme nach Anspruch 1,
**dadurch gekennzeichnet, dass**
das Wandelement (2, 2a) derart ausgerichtet ist, dass geräteseitig die konkav ausgebildete Seite (2c) des Abschnittes des Zylindermantels (2, 2a) und patientenseitig die konvex ausgebildete Seite (2d) des Abschnittes des Zylindermantels (2, 2a) angeordnet ist.

3. Trage-, Halte und Schutzvorrichtung für extrakorporale Herz- und/oder Lungenunterstützungssysteme nach einem der vorangegangenen Ansprüche,
**dadurch gekennzeichnet, dass**
die Halteeinrichtungen (3, 4) Halteelemente (9, 10) aufweisen, die in ihrer Geräteaufnahmegröße veränderbar sind, um Geräte mit unterschiedlichen Außenabmaßen aufnehmen zu können.

4. Trage-, Halte und Schutzvorrichtung für extrakorporale Herz- und/oder Lungenunterstützungssysteme nach Anspruch 3,
**dadurch gekennzeichnet, dass**
die Halteelemente (9, 10) zumindest teilweise ringförmig und/oder zylinderförmig und/oder greifbackenförmig ausgebildet sind, um zumindest ein Gerät (19, 20) für das Aufnehmen und Festhalten zu umschließen.

5. Trage-, Halte und Schutzvorrichtung für extrakorporale Herz- und/oder Lungenunterstützungssysteme nach einem der vorangegangenen Ansprüche,
**dadurch gekennzeichnet, dass**
die Halteeinrichtungen (3, 4) - in horizontaler Richtung betrachtet - nebeneinander angeordnet sind.

6. Trage-, Halte und Schutzvorrichtung für extrakorporale Herz- und/oder Lungenunterstützungssysteme nach einem der vorangegangenen Ansprüche
**dadurch gekennzeichnet, dass**
die Halteeinrichtungen (3, 4) mittels mindestens eines Haltesteges (5, 6) an der konkav ausgebildeten Seite (2c) des schirmartigen Wandelementes (2, 2a) befestigt sind, wobei in dem Haltesteg (5, 6) oder zwischen Haltesteg (5, 6) und Halteeinrichtung (3, 4) und/oder Wandelement (2) mindestens ein Gelenk (7), vorzugsweise ein Kugelgelenk, angeordnet ist, um die Halteinrichtungen (3, 4) in ihrer gegenseitigen Ausrichtung zueinander verändern zu können.

7. Trage-, Halte und Schutzvorrichtung für extrakorporale Herz- und/oder Lungenunterstützungssysteme nach einem der vorangegangenen Ansprüche,
**dadurch gekennzeichnet, dass**
in der Befestigungseinrichtung (15a, 15b, 16) oder zwischen der Befestigungseinrichtung (15a, 15b, 16) und der konvex ausgebildeten Seite (2d) des Wandelementes (2, 2a) mindestens ein Gelenk (16), vorzugsweise ein Kugelgelenk, angeordnet ist, um das Wandelement (2, 2a) und die Halteeinrichtungen (3, 4) gegenüber dem Gegenstand, wie ein Patientenbett (17), oder dem Patienten selbst ausrichten zu können.

8. Trage-, Halte und Schutzvorrichtung für extrakorporale Herz- und/oder Lungenunterstützungssysteme nach einem der vorangegangenen Ansprüche,
**dadurch gekennzeichnet, dass**
die Halteeinrichtungen (3, 4) derart ausgestaltet sind, dass mit ihnen Oxygenatoren, Blutpumpen, Blasenfallen sowie Steuerungseinrichtungen für temporäre oder langzeitextrakorporale Unterstützungssysteme jeglicher Art und Größe umfassbar und/oder festhaltbar sind.

9. Trage-, Halte und Schutzvorrichtung für extrakorporale Herz- und/oder Lungenunterstützungssysteme nach einem der vorangegangenen Ansprüche,
**dadurch gekennzeichnet, dass**
das Wandelement (2, 2a) auf Höhe der Kontaktpunkte der Haltestege (5, 6) mit der konkav ausgebildeten Seite (2c) des Wandelementes (2, 2a) ein zumindest teilweise kreisförmiges Versteifungselement (21) aufweist.

10. Trage-, Halte und Schutzvorrichtung für extrakorporale Herz- und/oder Lungenunterstützungssysteme nach einem der vorangegangenen Ansprüche,
**dadurch gekennzeichnet, dass**
das Wandelement (2, 2a) aus einem mechanisch und thermisch beanspruchbaren Material, wie beispielsweise ein Metall oder eine Metalllegierung und/oder eine Kunststoffzusammensetzung zumindest teilweise besteht.

## Claims

1. Supporting, retaining and protective device for extracorporeal heart and/or lung support systems, in particular for apparatuses for maintaining blood circulation, such as a heartlung machine, comprising a plurality of retaining units (3, 4) which are arranged on the apparatus side and are intended for securing blood-conducting and/or blood-treating apparatuses (19, 20) and control units,
and comprising at least one fastening unit (15a, 15b, 16) which is arranged on the patient side and is intended for detachably and rapidly fastening the supporting device (1) to a patient or an object (17) which is arranged near the patient,
**characterised in that**
at least one screen-like wall element (2, 2a) is arranged between the apparatus-side retaining units (3, 4) and the patient-side fastening unit (15a, 15b, 16) as a protective element for the apparatuses (19, 20) which are attached behind the wall element, which has the form of a portion of a cylinder jacket at least in part, a longitudinal axis (2, 2b) of the cylinder jacket (2, 2a), which is accessible from behind, extending in the vertical direction.

2. Supporting, retaining and protective device for extracorporeal heart and/or lung support systems according to claim 1, **characterised in that** the wall element (2, 2a) is oriented in such a way that the concave side (2c) of the portion of the cylinder jacket (2, 2a) is arranged on the apparatus side and the convex side (2d) of the portion of the cylinder jacket (2, 2a) is arranged on the patient side.

3. Supporting, retaining and protective device for extracorporeal heart and/or lung support systems according to any of the preceding claims, **characterised in that** the retaining units (3, 4) have retaining elements (9, 10) which can be modified in terms of the size of the apparatuses they receive, in order to be able to receive apparatuses having different external dimensions.

4. Supporting, retaining and protective device for extracorporeal heart and/or lung support systems according to claim 3, **characterised in that** the retaining elements (9, 10) are annular and/or cylindrical and/or in the form of a clamping jaw at least in part, in order to enclose at least one apparatus (19, 20) so as to receive and secure it.

5. Supporting, retaining and protective device for extracorporeal heart and/or lung support systems according to any of the preceding claims, **characterised in that** the retaining units (3, 4), when viewed in a horizontal direction, are arranged next to one another.

6. Supporting, retaining and protective device for extracorporeal heart and/or lung support systems according to any of the preceding claims, **characterised in that** the retaining units (3, 4) are fastened to the concave side (2c) of the screen-like wall element (2, 2a) by means of at least one retaining bar (5, 6), at least one joint (7), preferably a ball-and-socket joint, being arranged in the retaining bar (5, 6) or between the retaining bar (5, 6) and the retaining unit (3, 4) and/or the wall element (2) in order to be able to change the mutual orientation of the retaining units (3, 4).

7. Supporting, retaining and protective device for extracorporeal heart and/or lung support systems according to any of the preceding claims, **characterised in that** at least one joint (16), preferably a ball-and-socket joint, is arranged in the fastening unit (15a, 15b, 16) or between the fastening unit (15a, 15b, 16) and the convex side (2d) of the wall element (2, 2a) in order to be able to orient the wall element (2, 2a) and the retaining units (3, 4) relative to the object, such as a patient's bed (17), or the patient themselves.

8. Supporting, retaining and protective device for extracorporeal heart and/or lung support systems according to any of the preceding claims, **characterised in that** the retaining units (3, 4) are designed in such a way that oxygenators, blood pumps, bubble traps and control units for temporary or long-term extracorporeal support systems of any type and size can be included therein and/or secured thereby.

9. Supporting, retaining and protective device for extracorporeal heart and/or lung support systems according to any of the preceding claims, **characterised in that** the wall element (2, 2a) has, at the level of the points of contact of the retaining bars (5, 6) with the concave side (2c) of the wall element (2, 2a), a stiffening element (21) which is circular at least in part.

10. Supporting, retaining and protective device for extracorporeal heart and/or lung support systems according to any of the preceding claims, **characterised in that** the wall element (2, 2a) consists at least in part of a material that can withstand mechanical and thermal stresses, such as a metal or a metal alloy and/or a plastics composition.

## Revendications

1. Dispositif de support, de retenue et de protection pour des systèmes extracorporels d'assistance cardiaque et/ou pulmonaire, en particulier pour des appareils destinés à maintenir la circulation sanguine, tels qu'une machine coeur-poumon, comportant une pluralité de dispositifs de retenue (3, 4), disposés du côté des appareils, pour maintenir les appareils (19, 20) qui assurent la circulation du sang et/ou le traitement du sang et des dispositifs de commande,
- et comportant au moins un dispositif de fixation (15a, 15b, 16), disposé du côté du patient et destiné à la fixation amovible et rapide du dispositif de support (1) sur un patient ou sur un objet (17) disposé à proximité du patient, **caractérisé en ce qu'**
- entre les dispositifs de retenue (3, 4) du côté des appareils et le dispositif de fixation (15a, 15b, 16) du côté du patient est disposé au moins un élément de paroi (2, 2a) du type écran formant un élément de protection pour les appareils (19, 20) agencés derrière ledit élément de paroi qui possède, au moins en partie, la forme d'un tronçon d'une paroi cylindrique, un axe longitudinal (2, 2b) de la paroi cylindrique (2, 2a), accessible de l'arrière, s'étendant dans la direction verticale.

2. Dispositif de support, de retenue et de protection pour des systèmes extracorporels d'assistance cardiaque et/ou pulmonaire selon la revendication 1, **caractérisé en ce que** l'élément de paroi (2, 2a) est orienté de telle sorte que la face concave (2c) du tronçon de paroi cylindrique (2, 2a) est disposée du côté des appareils, et la face convexe (2d) du tronçon de paroi cylindrique (2, 2a) est disposée du côté du patient.

3. Dispositif de support, de retenue et de protection pour des systèmes extracorporels d'assistance cardiaque et/ou pulmonaire selon l'une quelconque des revendications précédentes, **caractérisé en ce que** les dispositifs de retenue (3, 4) comportent des éléments de retenue (9, 10), dont la dimension du logement de réception des appareils est modifiable, afin de pouvoir recevoir des appareils avec des dimensions extérieures différentes.

4. Dispositif de support, de retenue et de protection pour des systèmes extracorporels d'assistance cardiaque et/ou pulmonaire selon la revendication 3, **caractérisé en ce que** les éléments de retenue (9, 10) sont réalisés au moins en partie en forme d'anneau et/ou en forme de cylindre et/ou en forme de mâchoires de serrage pour enserrer au moins un appareil (19, 20), afin de le recevoir et de le maintenir.

5. Dispositif de support, de retenue et de protection pour des systèmes extracorporels d'assistance cardiaque et/ou pulmonaire selon l'une quelconque des revendications précédentes, **caractérisé en ce que** les dispositifs de retenue (3, 4) - considérés dans la direction horizontale - sont disposés l'un à côté de l'autre.

6. Dispositif de support, de retenue et de protection pour des systèmes extracorporels d'assistance cardiaque et/ou pulmonaire selon l'une quelconque des revendications précédentes, **caractérisé en ce que** les dispositifs de retenue (3, 4) sont fixés, au moyen d'au moins une tige de retenue (5, 6), sur la face concave (2c) de l'élément de paroi (2, 2a) du type écran, au moins une articulation (7), de préférence un joint sphérique, étant agencée dans la tige de retenue (5, 6) ou entre la tige de retenue (5, 6) et le dispositif de retenue (3, 4) et/ou l'élément de paroi (2), afin de pouvoir modifier l'orientation réciproque des dispositifs de retenue (3, 4).

7. Dispositif de support, de retenue et de protection pour des systèmes extracorporels d'assistance cardiaque et/ou pulmonaire selon l'une quelconque des revendications précédentes, **caractérisé en ce qu'**au moins une articulation (16), de préférence un joint sphérique, est agencée dans le dispositif de fixation (15a, 15b, 16) ou entre le dispositif de fixation (15a, 15b, 16) et la face convexe (2d) de l'élément de paroi (2, 2a), afin de pouvoir orienter l'élément de paroi (2, 2a) et les dispositifs de retenue (3, 4) par rapport à un objet, tel qu'un lit (17) d'un patient, ou par rapport au patient lui-même.

8. Dispositif de support, de retenue et de protection pour des systèmes extracorporels d'assistance cardiaque et/ou pulmonaire selon l'une quelconque des revendications précédentes, **caractérisé en ce que** les dispositifs de retenue (3, 4) sont configurés de telle sorte qu'ils permettent d'enserrer et/ou de maintenir des oxygénateurs, des pompes à sang, des pièges à bulles, et des dispositifs de commande pour des systèmes d'assistance extracorporels temporaires ou permanents de toute nature et grandeur.

9. Dispositif de support, de retenue et de protection pour des systèmes extracorporels d'assistance cardiaque et/ou pulmonaire selon l'une quelconque des revendications précédentes, **caractérisé en ce que** l'élément de paroi (2, 2a), à la hauteur des points de contact des tiges de retenue (5, 6) avec la face concave (2c) de l'élément de paroi (2, 2a), comporte un élément de raidissement (21) au moins partiellement circulaire.

10. Dispositif de support, de retenue et de protection pour des systèmes extracorporels d'assistance cardiaque et/ou pulmonaire selon l'une quelconque des revendications précédentes, **caractérisé en ce que** l'élément de paroi (2, 2a) est réalisé au moins en partie dans un matériau résistant aux sollicitations mécaniques et thermiques, tel qu'un métal ou un alliage métallique et/ou une composition de matière plastique.
